# EUROPÄISCHE PATENTSCHRIFT

(11) **EP 1 585 718 B1**
(45) Veröffentlichungstag und Bekanntmachung des Hinweises auf die Patenterteilung: **18.07.2007**
(21) Anmeldenummer: 03773653.5
(22) Anmeldetag: 18.10.2003
(51) Int. Cl.: C07C 213/00, C07C 215/60

(54) **VERFAHREN ZUR HERSTELLUNG VON (R)-SALBUTAMOL**
METHOD FOR THE PRODUCTION OF (R)-SALBUTAMOL
PROCEDE DE PRODUCTION DE (R)-SALBUTAMOL

(30) Priorität: 24.10.2002 DE 10249576
(43) Veröffentlichungstag der Anmeldung: 19.10.2005
(73) Patentinhaber: Boehringer Ingelheim Pharma GmbH & Co.KG, 55216 Ingelheim am Rhein (DE)
(72) Erfinder: KREYE, Paul, 55218 Ingelheim (DE); LENHART, Alfons, 55413 Weiler (DE); KLINGLER, Franz, Dietrich, 64347 Griesheim (DE)
(86) Internationale Anmeldenummer: PCT/EP2003/011583
(87) Internationale Veröffentlichungsnummer: WO 2004/037767

(56) Entgegenhaltungen:
- WO-A-00/43345
- WO-A-95/29146
- SAKURABA S ET AL: "EFFICIENT ASYMMETRIC HYDROGENATION OF ALPHA-AMINO KETONE DERIVATIVES A HIGHLY ENANTIOSELECTIVE SYNTHESIS OF PHENYLEPHRINE, LEVAMISOLE, CARNITINE AND PROPRANOLOL" CHEMICAL AND PHARMACEUTICAL BULLETIN, PHARMACEUTICAL SOCIETY OF JAPAN. TOKYO, JP, Bd. 43, Nr. 5, 1. Mai 1995 (1995-05-01), Seiten 738-747, XP000571426 ISSN: 0009-2363

## Beschreibung

Die vorliegende Erfindung betrifft ein verbessertes Verfahren zur Herstellung von (R)-Salbutamol mittels Rhodium-katalysierter asymmetrischer Hydrierung im industriellen Maßstab.

### Technologischer Hintergrund der Erfindung

(R)-Salbutamol, Levosalbutamol bzw. (R)-Albuterol ist α-[[(1,1-Dimethylethyl)-amino]methyl-4-hydroxy-1,3-benzenedimethanol gehört zu den pharmazeutisch als Bronchodilatoren eingesetzten β-2-Agonisten und ist von hohem kommerziellem Interesse. Die chemische Struktur des chiralen α-Aminoalkohols (R)-Salbutamol ist in der Formel I dargestellt:

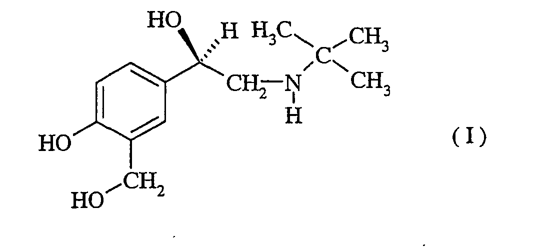

### Stand der Technik

Zu den aus dem Stand der Technik bekannten Herstellungsverfahren von (R)-Salbutamol gehört die Racematspaltung des racemischen Salbutamols mit Hilfe von di-Toluylweinsäure, z.B. gemäß des US Patentes US 5,399,765. Weiterhin schlägt die internationale Patentanmeldung WO 95/29146 die Herstellung von (R)-Salbutamol ausgehend vom entsprechenden alpha-Iminoketon durch enantioselektive Reduktion mit Boranen in Gegenwart eines chiralen Oxaborazol Katalysators vor.

Zur Herstellung von (R)-Salbutamol in industriellem Maßstab sind jedoch die im Stand der Technik beschriebenen Verfahren nicht geeignet, da bei der Racematspaltung die Hälfte des wertvollen Ausgangsprodukts verloren geht bzw. bei der entantioselektiven Reduktion große Mengen des schwerzugänglichen Oxaborazol Katalysators eingesetzt werden müssen.

Eines der wesentlichen Ziele der vorliegenden Erfindung ist es, ein Verfahren zu entwickeln, durch das (R)-Salbutamol in hoher optischer und chemischer Reinheit hergestellt werden kann. Damit soll z.B. die Gefahr einer Verunreinigung von Arzneistoffen, die (R)-Salbutamol als Wirkstoff enthalten, mit dem unerwünschten D-Enantiomeren minimiert werden.

Ein weiteres Ziel der Erfindung besteht darin, ein Verfahren zu entwickeln, durch das weitgehend enantiomerenreines (R)-Salbutamol ausgehend von leicht zugänglichen Ausgangsmaterialien in einfacher Weise dargestellt werden kann.

Ein weiteres Ziel der Erfindung besteht darin, (R)-Salbutamol über ein stereoselektives Verfahren herzustellen, um Reaktionsschritte zu vermeiden, bei denen chirale Zwischenverbindungen bzw. das chirale Endprodukt (R)-Salbutamol als Racemat in einer ähnlichen Menge wie der entsprechende Antipode anfällt.

Überraschenderweise wurde nun gefunden, dass man (R)-Salbutamol im technischen Maßstab in sehr guten Ausbeuten und guter optischer Reinheit erhalten kann, wenn man Salbutamon einer asymmetrischen Hydrierung in Gegenwart von Rhodium und einem chiralen, zweizähnigen Phosphinliganden als Katalysatorsystem unterwirft.

### Beschreibung der Erfindung

Die vorliegende Erfindung betrifft ein Verfahren zur Herstellung von Levosalbutamol bzw. (R)-Salbutamol oder der pharmakologisch akzeptablen Salze davon ausgehend von prochiralem Salbutamon als Edukt, wobei man Salbutamon einer asymmetrischen Hydrierung in Gegenwart von Rhodium und einem chiralen, zweizähnigen Phosphinliganden (PP*), insbesondere (2R, 4R)-4-(Dicyclohexylphosphino)-2-(diphenylphosphino-methyl)-N-methylaminocarbonyl-pyrrolidin, als Katalysatorsystem unterwirft, und das erhaltene Levosalbutamol gegebenenfalls mit einer Säure in ein Salz überführt.

Bevorzugt ist ein Verfahren, wobei die asymmetrische Hydrierung in einem Temperaturbereich von 20°C bis 100°C, vorzugsweise von 40°C bis 60°C, insbesondere von 45°C bis 55°C durchgeführt wird.

Weiterhin bevorzugt ist ein Verfahren, wobei die asymmetrische Hydrierung unter einem Druck von mehr als 1 bar bis 100 bar, vorzugsweise unter einem Druck von 10 bar bis 50 bar, insbesondere bei etwa 20 bar durchgeführt wird.

Als Reaktionsmedien können sowohl protische Lösungsmittel - wie z.B. Alkohole und/oder Wasser - oder aprotische polare Lösungsmittel wie z.B. Ether und/oder Amide bzw. Lactame und/oder Gemische davon eingesetzt werden. Allen Lösungsmitteln kann gegebenenfalls Wasser zugesetzt sein. Als protische Lösungsmittel werden bevorzugt verzweigte oder unverzweigte C₁ - C₈ Alkanole eingesetzt. Besonders bevorzugt werden niedere Alkohole wie Methanol, Ethanol, n-Propanol und Isopropanol oder deren Mischungen eingesetzt. Besonders bevorzugt wird als Reaktionsmedium Methanol verwendet, wobei das Methanol oder die anderen Alkohole oder Lösungsmittel gegebenenfalls Wasser enthalten kann (können). Als aprotische Lösungsmittel eignen sich polare Ether wie beispielsweise Tetrahydrofuran oder Dimethoxyethylether oder Amide wie beispielsweise Dimethylformamid, oder Lactame wie beispielsweise N-Methylpyrrolidon. Bevorzugt werden Lösungsmittel eingesetzt, die wenig zur Brennbarkeit neigen.

Die Reaktion wird vorzugsweise in Gegenwart einer Base durchgeführt. Als Base können organische Basen oder anorganische Basen sowohl als Feststoffe als auch in Form von Lösungen eingesetzt werden, z.B. als wäßrige Lösungen. Als anorganische Basen eignen sich basisch reagierende Alkalisalze oder Alkalihydroxyde. Bevorzugt werden Alkalihydrogencarbonate oder Alkalicarbonate neben Alaklihydroxiden eingesetzt. Ganz besonders bevorzugt werden Na₂CO₃, K₂CO₃, LiOH, NaOH, KOH oder NaHCO₃ verwendet.

Als organische Basen eignen sich tertiäre Amine, insbesondere tertiäre Alkyl-Amine, tertiäre Alkyl-Aryl-Amine oder Pyridine. Bevorzugt werden Trialkylamine mit verzweigten oder unverzweigten C₁- C₅-Alkylresten eingesetzt. Als ganz besonders bevorzugt haben sich beispielsweise Triethylamin oder Diisopropylethylamin bewährt. Gegebenenfalls kann die Reaktion auch in Gegenwart von basischen Polymeren mit z.B. tertiären Aminofunktionen durchgeführt werden.

Bevorzugt sind solche Verfahren, wobei man Salbutamon zum Rhodiumkatalysator bei der asymmetrischen Hydrierung in einem Mol-Verhältnis von 500:1 bis 100000:1, vorzugsweise von 750:1 bis 20000:1 einsetzt.

Bei einem Mol-Verhältnis Katalysator zu Substrat von etwa 1:1000 erhält man durch das erfindungsgemäße Verfahren ausgehend von Salbutamon (R)-Salbutamol bereits in einer optischen Reinheit von 70 % ee (Reaktionsschema 1). Durch Überführen des Salbutamols **(I)** in ein Säureadditionssalz und anschließendem Ausfällen derselben aus einem Amoniak-Methanol-Wasser-Gemisch kann die optische Reinheit in einfacher und bemerkenswerter Weise weiter gesteigert werden.

Weiter wurde gefunden, daß im Gegensatz zur Lehre der WO 95/29146 für die asymmetrische Reduktion zum Erzielen guter Ausbeuten bzw. hoher optischer Reinheit ein Mol-Verhältnis von Katalysator zu Substrat nicht - wie dort offenbart - von etwa 1:10 notwendig ist. Im erfindungsgemäßen Verfahren kann dieses Verhältnis drastisch um den Faktor 10 bis 1000 gesenkt werden. Trotz dieser signifikanten Verringerung der Katalysatormenge wird das aus der asymmetrischen Hydrierung hervorgehende (R)-Salbutamol dennoch in einer deutlich höheren optischen Ausbeute erhalten als durch das aus dem Stand der Technik bekannten Verfahren. Durch die Reduktion der Katalystormenge wird die Aufreinigung des Produktes deutlich vereinfacht.

Durch die Verringerung der Katalysatormenge und Verwendung des kommerziell günstigen Salbutamon als Edukt können die Kosten der Herstellung von (R)-Salbutamol durch das neue Verfahren deutlich gesenkt werden.

Das als Ausgangsprodukt einzusetzende Salbutamon erhält man durch Hydrierung von N-Benzylsalbutamon, welches durch Bromierung von 4-Acetyloxy-3-Acetyloxymethylbenzophenon und anschließender Umsetzung mit *tert*-Butyl-benzylamin (TBBA) herstellbar ist gemäß nachfolgendem Reaktionsschema:

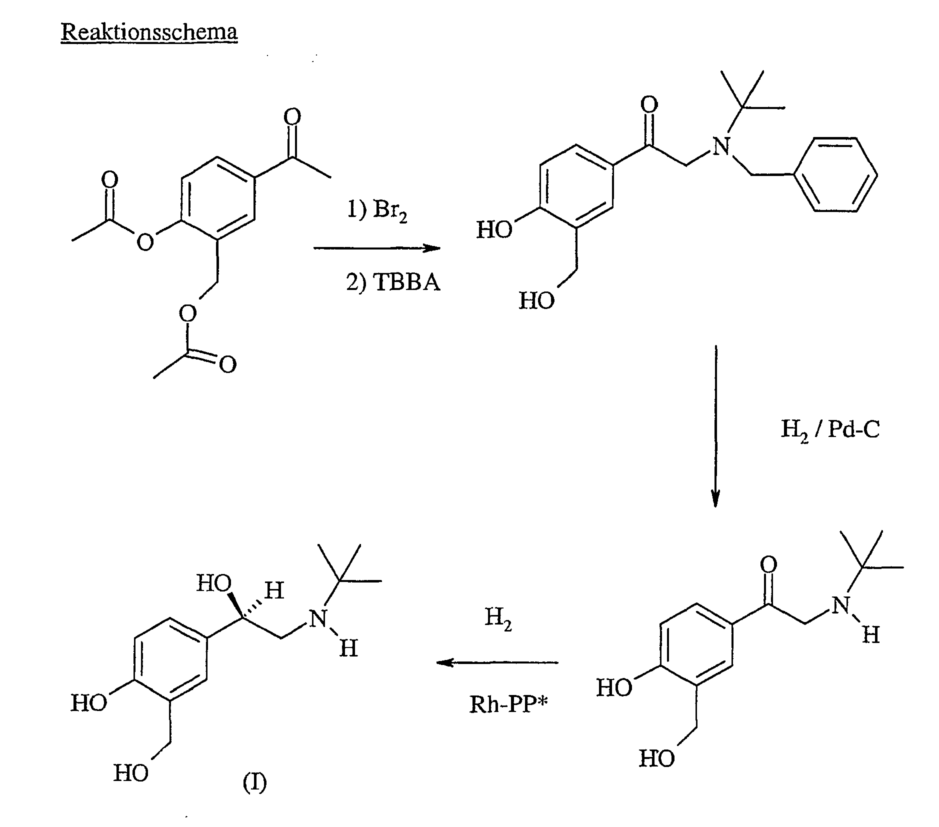

Zusätzlich konnte durch das neue Verfahren die Raum-Zeitausbeute gegenüber dem Stand der Technik verbessert werden. Letzteres ist gerade unter Kosten- und Sicherheitsgesichtspunkten für die Herstellung von (R)-Salbutamol in industriellem Maßstab besonders vorteilhaft.

Schließlich ist es gemäß dem erfindungsgemäßen Verfahren möglich, auf den Schutz der phenolischen Hydroxylgruppe in Salbutamon zu verzichten und dabei trotzdem erfolgreich zu dem chiralen (R)-Salbutamol unter Anwendung einer asymmetrischen Hydrierung mit einem der erfindungsgemäßen Katalysatorsysteme umzusetzen.

Als Katalysator wird erfindungsgemäß [Rh(COD)Cl]₂, wobei COD für eine Cyclooctadienylgruppe steht, und ein chiraler, zweizähniger Phosphinligand (PP*) eingesetzt. Bevorzugt wird (2R, 4R)-4-(Dicyclohexylphosphino)-2-(diphenylphosphino-methyl)-N-methyl-aminocarbonylpyrrolidin (RR-MCCPM) als Katalysator eingesetzt.

Die Herstellung dieses Katalysators ist aus dem Stand der Technik bekannt [EP-A-0 251 164, EP-A-0 336 123]. Der Katalysator kann auch polymergebunden vorliegen, z.B. indem der chirale Ligand (2R, 4R)-4-Dicyclohexylphosphino)-2-(diphenylphosphino-methyl)-N-methylaminocarbonyl) pyrrolidin z.B. über die Phenylgruppen an ein Polymer gebunden ist. Dabei schließt die Verwendung solcher polymergebundener Liganden den gleichzeitigen Einsatz von nicht-polymergebundenem Liganden nicht zwingend aus. Solche polymergebunden Katalysatoren sind insbesondere für eine einfache Reinigung des Produktes von Vorteil.

Der Katalysator wird entweder als vorgefertigte, sauerstofffreie Lösung von [Rh(COD)Cl]₂ und Ligand eingesetzt oder *in situ* aus [Rh(COD)Cl]₂ und Ligand in Gegenwart von Salbutamon sauerstofffrei unter Schutzgasatmosphäre oder Wasserstoffatmosphäre hergestellt.

Die Hydrierung wird in der Regel sauerstofffrei, zweckmäßigerweise unter Inertgas, durchgeführt, bevorzugt unter Wasserstoffatmosphäre. Für die Reaktion ist es jedoch nicht zwingend, daß der Wasserstoff für die Hydrierung aus dem Atmosphärengas über der Reaktionsmischung entnommen werden kann. Der Wasserstoff kann auch *in situ* in Lösung aus geeigneten Wasserstoffquellen erzeugt werden. Zu solchen Wasserstoffquellen zählen z.B. Ammoniumformiat, Ameisensäure und andere Formiate, Hydrazine in Gegenwart von Metallionen wie Fe²⁺/Fe³⁺ und andere aus dem Stand der Technik bekannte Wasserstoffquellen.

Die Reaktionszeit für die asymmetrische Hydrierung beträgt in der Regel bis zu ihrer Beendigung zwischen 2 und 48 Stunden, bevorzugt liegt sie zwischen 4 und 36 Stunden, besonders bevorzugt beträgt sie etwa 23 Stunden.

Die Umsetzung von N-Benzyl-Salbutamon zum Salbutamon gelingt durch Palladiumkatalysierte hydrierende Debenzylierung. Dabei kann die Reaktionsmischung der asymmetrischen Hydrierung ohne weitere Aufarbeitung mit einem Palladium-Katalysator versetzt werden.

Bei dieser Methode wird Benzylsalbutamon mit Aktivkohle und einer Palladiumchlorid-Lösung versetzt und unter einem Druck von größer 1 bis 5 bar, bevorzugt bei 2 - 3 bar, hydriert. Die weitere Aufarbeitung erfolgt nach literaturbekannten Verfahren.

Das erfindungsgemäße Verfahren soll nun durch die nachfolgenden Beispiele erläutert werden. Dem Fachmann ist bewußt, daß die Beispiele nur zur Veranschaulichung dienen und als nicht limitierend anzusehen sind.

### Beispiele

### Beispiel 1 Benzylsalbutamon

900 g 4-Acetyloxy-3-acetyloxymethylbenzophenon werden in 61 geeignetem Lösungsmittel vorgelegt. Nach Zugabe von 614 g Brom wird noch 30 Minuten am Rückfluss gekocht und abgekühlt. Nach Zugabe von 1153 g *tert*-Butylbenzylamin wird erneut 20-25 h am Rückfluss gekocht. Nach Filtration des Niederschlages wird die organische Phase mit Salzsäure extrahiert und das Produkt kristallisiert. Man erhält 806 g Benzylsalbutamon.

### Beispiel 2 Salbutamon:

36,4 g Benzylsalbutamon werden in 110 ml Wasser aufgenommen und mit 1 g Pd/C, 10%ig, bei 2 bar Wasserstoffdruck und 40 °C für 2,5 gerührt. Der Niederschlag wird mit Methanol in Lösung gebracht. Die Lösung wird über Celite filtriert und eingeengt bis zur beginnenden Kristallisation. Man kühlt über Nacht auf Raumtemperatur und filtriert die Kristalle ab. Nach Waschen mit wenig kaltem Wasser und Trocknen über Nacht bei 50 °C im Vakuum erhält man Salbutamon in 92% Ausbeute.

### Beispiel 3 (R)-(-)-Salbutamol

10 g Salbutamon werden in 100 ml Methanol (entgast) und 0,13 ml Triethylamin gelöst. Man gibt 4,7 mg (RhCODCl)2 und 10 mg (2R, 4R)-4-Dicyclohexylphosphino)-2-(diphenylphosphino-methyl)-N-methyl-aminocarbonyl) pyrrolidin (als toluolische Lösung) zu und rührt 23 h bei 50 °C und 20 bar Wasserstoffdruck. Die Reaktionslösung wird einrotiert und der Rückstand aus Ethanol umkristallisiert. Man erhält das Salbutamol in einer Ausbeute von 90% in einer optischen Reinheit von ca. 70% e.e.

## Patentansprüche

1. Verfahren zur Herstellung von Levosalbutamol oder der pharmakologisch akzeptablen Salze davon ausgehend von prochiralem Salbutamon als Edukt, **dadurch gekennzeichnet, dass** man Salbutamon einer asymmetrischen Hydrierung in Gegenwart von Rhodium und einem chiralen, zweizähnigen Phosphinliganden als Katalysatorsystem unterwirft, und das erhaltene Levosalbutamol gegebenenfalls mit einer Säure in ein Salz überführt.

2. Verfahren nach Anspruch 1, **dadurch gekennzeichnet dass** der Ligand (2R, 4R)-4-(Dicyclohexylphosphino)-2-(diphenylphosphina-methyl)-N-methyl-aminocarbonylpyrrolidin oder polymergebundenes (2R, 4R)-4-(Dicyclohexylphosphino)-2-(diphenylphosphino-methyl)-N-methyl-aminocarbonyl-pyrrolidin ist.

3. Verfahren nach einem der Ansprüche 1 bis 2, **dadurch gekennzeichnet, dass** die asymmetrische Hydrierung in einem Temperaturbereich von 20°C bis 100°C durchgeführt wird.

4. Verfahren nach einem der vorangegangenen Ansprüche 1 bis 3, **dadurch gekennzeichnet, dass** die asymmetrische Hydrierung unter einem Druck von mehr als 1 bar bis 100 bar, vorzugsweise unter einem Druck von 10 bar bis 50 bar durchgeführt wird.

5. Verfahren nach einem der vorangehenden Ansprüche 1 bis 4, **dadurch gekennzeichnet, dass** die asymmetrische Hydrierung in einem protischen Lösungsmittel durchgeführt wird.

6. Verfahren nach Anspruch 5, **dadurch gekennzeichnet, dass** die asymmetrische Hydrierung in einem verzweigten oder unverzweigten C₁ - C₈-Alkanol als Lösungsmittel durchgeführt wird.

7. Verfahren nach einem der vorangegangenen Ansprüche 5 oder 6, **dadurch gekennzeichnet, dass** das Lösungsmittel für die asymmetrische Hydrierung Wasser enthält.

8. Verfahren nach einem der vorangegangenen Ansprüche 1 bis 7, **dadurch gekennzeichnet, dass** man Salbutamon zum Rhodiumkatalysator bei der asymmetrischen Hydrierung in einem Mol-Verhältnis von 500:1 bis 100000;1, vorzugsweise von 750:1 bis 20000:1 einsetzt.

9. Verfahren nach einem der vorangegangenen Ansprüche 1 bis 8, **dadurch gekennzeichnet, dass** der Rhodiumkatalysator für die asymmetrische Hydrierung als vorgefertigte Lösung eingesetzt wird.

10. Verfahren nach einem der vorangegangenen Ansprüche 1 bis 8, **dadurch gekennzeichnet, dass** der Rhodiumkatalysator für die asymmetrische Hydrierung *in situ* erzeugt wird.

11. Verfahren nach einem der vorangegangenen Ansprüche 1 bis 10, **dadurch gekennzeichnet, dass** die asymmetrische Hydrierung innerhalb einer Reaktionszeit von 2 bis 48 Stunden, vorzugsweise von 4 bis 36 Stunden durchgeführt wird.

12. Verfahren nach einem der vorangegangenen Ansprüche 1 bis 11, **dadurch gekennzeichnet, dass** Salbutamon ausgehend von N-Benzylsalbutamon durch Hydrierung in Gegenwart eines Palladiumkatalysators hergestellt wird.

13. Verfahren zur Herstellung von Levosalbutamol oder der pharmakologisch akzeptablen Salze davon, welches die folgenden Schritte umfasst:
(a) Bromierung von 4-Acetylox-3-acetyloxymethylbenzophenon,
(b) Umsetzung des erhaltenen Produkts mit N-*tert*-Butyl-N-benzylamin,
(c) Hydrierung des erhaltenen N-Benzylsalbutamon in Gegenwart eines Palladiumkatalysators,
(d) Hydrierung des erhaltenen Salbutamons in Gegenwart von Rhodium und einem chiralen, zweizähnigen Phosphinliganden, und
gegebenenfalls Behandlung mit einer Säure.

## Claims

1. Process for preparing levosalbutamol or the pharmacologically acceptable salts thereof starting from prochiral salbutamone as educt, **characterised in that** salbutamone is subjected to asymmetric hydrogenation in the presence of rhodium and a chiral bidentate phosphine ligand as catalyst system, and the levosalbutamol obtained is optionally converted into a salt with an acid.

2. Process according to claim 1, **characterised in that** the ligand is (2R, 4R)-4-(dicyclohexylphosphino)-2-(diphenylphosphino-methyl)-N-methylaminocarbonyl-pyrrolidine or polymer-bound (2R, 4R)-4-(dicyclohexylphosphino)-2-(diphenylphosphino-methyl)-N-methylaminocarbonyl-pyrrolidine.

3. Process according to one of claims 1 to 2, **characterised in that** the asymmetric hydrogenation is carried out in a temperature range from 20°C to 100°C.

4. Process according to one of the preceding claims 1 to 3, **characterised in that** the asymmetric hydrogenation is carried out under a pressure of more than 1 bar to 100 bar, preferably under a pressure of 10 bar to 50 bar.

5. Process according to one of the preceding claims 1 to 4, **characterised in that** the asymmetric hydrogenation is carried out in a protic solvent.

6. Process according to claim 5, **characterised in that** the asymmetric hydrogenation is carried out in a branched or unbranched C₁-C₈-alkanol as solvent.

7. Process according to one of the preceding claims 5 or 6, **characterised in that** the solvent for the asymmetric hydrogenation contains water.

8. Process according to one of the preceding claims 1 to 7, **characterised in that** during asymmetric hydrogenation salbutamone is used in a molar ratio to the rhodium catalyst of from 500:1 to 100000:1, preferably from 750:1 to 20000:1.

9. Process according to one of the preceding claims 1 to 8, **characterised in that** the rhodium catalyst for the asymmetric hydrogenation is used as a pre-prepared solution.

10. Process according to one of the preceding claims 1 to 8, **characterised in that** the rhodium catalyst for the asymmetric hydrogenation is produced *in situ.*

11. Process according to one of the preceding claims 1 to 10, **characterised in that** the asymmetric hydrogenation is carried out within a reaction time of 2 to 48 hours, preferably 4 to 36 hours.

12. Process according to one of the preceding claims 1 to 11, **characterised in that** salbutamone is prepared starting from N-benzylsalbutamone by hydrogenation in the presence of a palladium catalyst.

13. Process for preparing levosalbutamol or the pharmacologically acceptable salts thereof, which comprises the following steps:
(a) brominating 4-acetyloxy-3-acetyloxymethylbenzophenone,
(b) reacting the product obtained with N-*tert*-butyl-N-benzylamine,
(c) hydrogenating the N-benzylsalbutamone obtained in the presence of a palladium catalyst,
(d) hydrogenating the salbutamone obtained in the presence of rhodium and a chiral bidentate phosphine ligand, and
optionally treating with an acid.

## Revendications

1. Procédé de production du lévosalbutamol ou de sels pharmacologiquement acceptables de celui-ci à partir de salbutamone prochirale comme produit de départ, **caractérisé en ce que** l'on soumet la salbutamone à une hydrogénation asymétrique en présence de rhodium et d'un ligand phosphine bidenté chiral comme système de catalyseur, et on convertit éventuellement le lévosalbutamol obtenu en un sel avec un acide.

2. Procédé selon la revendication 1 **caractérisé en ce que** le ligand est la (2R,4R)-4-(dicyclohexylphosphino)-2-(diphénylphosphino-méthyl)-N-méthylaminocarbonyl-pyrrolidine ou la (2R,4R)-4-(dicyclohexylphosphino)-2-(diphénylphosphino-méthyl)-N-méthyl-aminocarbonyl-pyrrolidine liée à un polymère.

3. Procédé selon l'une des revendications 1 à 2 **caractérisé en ce que** l'hydrogénation asymétrique est conduite dans un domaine de température de 20°C à 100°C.

4. Procédé selon l'une des revendications 1 à 3 précédentes **caractérisé en ce que** l'hydrogénation asymétrique est conduite sous une pression de plus de 1 bar à 100 bar, de préférence sous une pression de 10 bar à 50 bar.

5. Procédé selon l'une des revendications 1 à 4 précédentes **caractérisé en ce que** l'hydrogénation asymétrique est conduite dans un solvant protique.

6. Procédé selon la revendication 5 **caractérisé en ce que** l'hydrogénation asymétrique est conduite dans un C₁-C₈-alcanol ramifié ou non ramifié comme solvant.

7. Procédé selon l'une des revendications 5 ou 6 précédentes **caractérisé en ce que** le solvant pour l'hydrogénation asymétrique contient de l'eau.

8. Procédé selon l'une des revendications 1 à 7 précédentes **caractérisé en ce que** l'on utilise la salbutamone lors de l'hydrogénation asymétrique dans un rapport molaire au catalyseur au rhodium de 500 : 1 à 100000 : 1, de préférence de 750 : 1 à 20000 : 1.

9. Procédé selon l'une des revendications 1 à 8 précédentes **caractérisé en ce que** le catalyseur au rhodium pour l'hydrogénation asymétrique est utilisé sous forme de solution préparée au préalable.

10. Procédé selon l'une des revendications 1 à 8 précédentes **caractérisé en ce que** le catalyseur au rhodium pour l'hydrogénation asymétrique est produit *in situ.*

11. Procédé selon l'une des revendications 1 à 10 précédentes **caractérisé en ce que** l'hydrogénation asymétrique est conduite en une durée de réaction de 2 à 48 heures, de préférence de 4 à 36 heures.

12. Procédé selon l'une des revendications 1 à 11 précédentes **caractérisé en ce que** la salbutamone est produite à partir de la N-benzylsalbutamone par hydrogénation en présence d'un catalyseur au palladium.

13. Procédé de production du lévosalbutamol ou de sels pharmacologiquement acceptables de celui-ci qui comprend les étapes suivantes :
(a) bromation de la 4-acétyloxy-3-acétyloxyméthylbenzophénone,
(b) réaction du produit obtenu avec la N-*tert*-butyl-N-benzylamine,
(c) hydrogénation de la N-benzylsalbutamone obtenue en présence d'un catalyseur au palladium,
(d) hydrogénation de la salbutamone obtenue en présence de rhodium et d'un ligand phosphine bidenté chiral, et
éventuellement traitement avec un acide.
